# DEMANDE DE BREVET EUROPEEN

(11) **EP 3 679 928 A1**
(43) Date de publication de la demande: **15.07.2020**
(21) Numéro de dépôt: 19305019.2
(22) Date de dépôt: 08.01.2019
(51) Int. Cl.: A61K 9/51, A61K 9/06, A61K 38/12

(54) **COMPOSITION PHARMACEUTIQUE DE TYPE GEL POUR TRAITER/PREVENIR UNE INFECTION**

(71) Demandeur: Atlangram, 44200 Nantes (FR)
(72) Inventeur: MEYER, Olivier, 49000 Angers (FR); REGHAL, Amokrane, 44260 Savenay (FR)
(74) Mandataire: Nony

(57) **Abrégé**

La présente invention concerne une composition pharmaceutique stabilisée à un état gélifié à au moins une température variant de 15 °C à 40 °C, comprenant au moins une phase aqueuse gélifiée avec au moins un gélifiant hydrophile polymérique, des nanocapsules lipidiques comprenant un coeur lipidique liquide ou semi-liquide à température ambiante enveloppé d'une enveloppe lipidique solide à température ambiante, lesdites phase aqueuse gélifiée et nanocapsules contenant au moins un antibiotique, identique ou différent, l'antibiotique dans ladite phase aqueuse y étant présent à l'état de soluté.

## Description

La présente invention concerne des nouvelles compositions pharmaceutiques, appropriées pour une administration localisée et contrôlée en termes de durée, de principes actifs à l'image des antibiotiques, ainsi que des dispositifs médicaux correspondants. Elle concerne en outre leurs utilisations thérapeutiques pour traiter ou prévenir une infection, notamment bactérienne et en particulier les infections bactériennes ostéo-articulaires.

Les antibiotiques demeurent aujourd'hui les principes actifs les plus utilisés pour traiter les infections bactériennes. Toutefois, dans certains cas, leur mise en oeuvre ne s'avère pas encore totalement satisfaisante notamment en termes d'efficacité, de spectre d'action et de biodisponibilité.

De plus, les antibiotiques sont généralement délivrés au patient par voie orale ou parentérale. Or, dans le cas d'infections bactériennes spécifiquement localisées, ce mode d'administration peut être insuffisamment efficace compte-tenu d'un défaut de diffusion optimale au niveau du site infectieux. Sont en particulier concernées par cette difficulté, les infections touchant les os, les articulations, ou des infections se développant au niveau d'un dispositif médical implanté, tel qu'un implant ou une prothèse, et pour lesquelles il est souvent nécessaire de mettre en oeuvre un traitement chirurgical supplémentaire afin de résorber l'infection.

En conséquence, la mise au point de formulations galéniques dédiées à l'administration d'actifs pour traiter des infections, en particulier bactériennes, qui soient plus performantes en termes de propriétés pharmacocinétiques et pharmacodynamiques et de ciblage relève d'un grand intérêt.

Il est déjà connu de FR3017294 de véhiculer des antibiotiques tels que la daptomycine, la vancomycine et la rifampicine dans des nanocapsules lipidiques. Cette forme galénique s'avère tout particulièrement intéressante sur le plan de la biodisponibilité et permet donc de mettre en oeuvre des doses plus réduites en antibiotiques pour une efficacité au moins équivalente à celle des autres formes. Toutefois, ces nanocapsules lipidiques ne tolèrent qu'un faible taux de charge en antibiotique, à savoir un taux inférieur à 50 mg/ml de suspension. En effet, au-delà d'une certaine concentration en antibiotique, les propriétés tensioactives de celui-ci perturbent la formation des particules. Par ailleurs, ces nanocapsules ne permettent pas de moduler dans le temps un profil de libération du principe actif. Le plus souvent, la libération est rapide et/ou non linéaire.

Enfin, l'administration de ces nanocapsules est pour l'essentiel préconisée par voie parentérale. En conséquence, la difficulté de cibler localement une infection bactérienne comme celles de type ostéo-articulaire demeure.

Or, les infections ostéo-articulaires sont des pathologies graves qui touchent un os et/ou une articulation. Elles peuvent survenir spontanément à la suite d'une septicémie, après une plaie profonde ou une fracture ouverte par exemple, ou bien constituer une complication d'un acte chirurgical, comme par exemple la pose d'un implant, prothèse, plaque ou vis, ce que l'on appelle communément les infections sur matériel étranger (ou dispositif médical implanté). Ces infections ostéo-articulaires demeurent donc encore aujourd'hui difficiles à traiter, surtout s'agissant des infections sur prothèses articulaires, car les bactéries se fixent également sur les matériaux des implants, rendant les antibiotiques peu efficaces et nécessitant un traitement chirurgical afin de contrôler ou résorber l'infection.

Il est en outre à souligner que le traitement des infections ostéo-articulaires peut être particulièrement éprouvant pour le patient en raison de la lourdeur et de la durée de l'antibiothérapie et avec trop souvent la nécessité de recourir à une chirurgie préalable adaptée afin de contrôler ou résorber l'infection suivie le plus souvent d'une période post-opératoire de rééducation.

La présente invention vise précisément à proposer une nouvelle forme galénique pour antibiotiques permettant de surmonter les insuffisances précitées.

En particulier, la présente invention propose une composition pharmaceutique efficace pour délivrer localement une quantité efficace d'au moins un actif thérapeutique, notamment un antibiotique, et en particulier directement au niveau du site siège d'infection bactérienne.

La présente invention vise en particulier à proposer une composition pharmaceutique appropriée à une administration ciblée d'au moins un actif thérapeutique, notamment un antibiotique pour traiter une infection bactérienne touchant les os, les articulations, ou se développant au niveau d'un dispositif médical implanté, tel qu'un implant ou une prothèse.

En particulier, la présente invention propose une composition pharmaceutique efficace pour traiter localement une infection bactérienne ostéo-articulaire.

La présente invention vise en outre à proposer une composition pharmaceutique efficace pour délivrer de manière contrôlée et notamment prolongée dans le temps le principe actif qu'elle contient de manière à ce que le médicament soit le plus efficace possible et possède une durée d'action prolongée.

Ainsi, la présente invention concerne à titre principal, une composition pharmaceutique stabilisée à un état gélifié à au moins une température variant de 15 °C à 40 °C, comprenant au moins :
- une phase aqueuse gélifiée avec au moins un gélifiant hydrophile polymérique,
- des nanocapsules lipidiques comprenant un coeur lipidique liquide ou semi-liquide à température ambiante enveloppé d'une enveloppe lipidique solide à température ambiante, lesdites phase aqueuse gélifiée et nanocapsules contenant au moins un antibiotique, identique ou différent, l'antibiotique dans ladite phase aqueuse y étant présent à l'état de soluté.

En particulier, ledit ou lesdits gélifiants hydrophiles polymériques sont choisis parmi les carboxyalkylcelluloses en C₁-C₂ possédant une viscosité supérieure à 1500 mPa.s, les copolymères tribloc constitués de poly(oxyde d'éthylène) et de poly(oxyde de propylène), les copolymères tribloc constitués de poly(éthylène glycol) et de poly(acide lactique-co-glycolique), et leurs mélanges.

Plus particulièrement, les copolymères tribloc constitués de poly(oxyde d'éthylène) et de poly(oxyde de propylène) peuvent être thermosensibles et présenter une température critique inférieure de solubilité (LCST) comprise entre 15 °C et 40 °C, et les copolymères tribloc constitué de poly(éthylène glycol) et de poly(acide lactique-co-glycolique) peuvent être thermosensibles et présenter une température critique inférieure de solubilité (LCST) comprise entre 15 °C et 40 °C.

De manière surprenante, les inventeurs ont ainsi constaté que la formulation de nanocapsules lipidiques spécifiques contenant au moins un antibiotique, au sein d'une phase aqueuse gélifiée conforme à l'invention et elle-même chargée en antibiotique s'avère tout particulièrement avantageuse.

Contre toute attente, le conditionnement des nanocapsules chargées en antibiotique, dans une phase aqueuse gélifiée n'est pas préjudiciable d'une part à l'intégrité des nanocapsules lors du stockage de la composition pharmaceutique ni à la libération *in vivo* de l'antibiotique contenu dans lesdites nanocapsules.

Une composition pharmaceutique conforme à l'invention s'avère en outre adaptée pour une administration localisée et donc à proximité voire au contact d'un organe ou implant *in vivo.* Ceci est particulièrement intéressant pour prévenir et/ou traiter les infections ostéo-articulaires.

Au regard de sa viscosité, elle peut être aisément prélevée et appliquée au niveau des sites infectieux ou susceptibles d'être infectés ou de l'implant dédié à être en contact ou déjà au contact de ce site.

Une composition pharmaceutique conforme à l'invention est propice à la formulation d'une quantité élevée en antibiotique non accessible avec uniquement des nanocapsules lipidiques conventionnelles.

Elle autorise en outre une libération contrôlée et notamment prolongée en antibiotique dans la mesure où elle véhicule celui ou ceux-ci selon deux modes distincts, l'un directement dans la phase aqueuse gélifiée et l'autre sous une forme encapsulée dans la phase aqueuse gélifiée. Ceci est particulièrement intéressant pour le traitement et/ou la prévention d'infections internes comme les infections ostéo-articulaires. Il n'est pas nécessaire de procéder à des administrations répétées sous bref délai pour garantir une efficacité des antibiotiques. En particulier, les compositions selon l'invention permettent de libérer de façon constante le principe actif sur une période d'au moins 14 jours. A l'issue d'une période de 14 jours, les concentrations encore présentes en antibiotique restent très élevées, dépassant de 50 à 100 fois les Concentrations Minimales Inhibitrices (CMI) des bactéries généralement à l'origine de ces infections.

La présente invention concerne en outre un dispositif médical interne dédié à être manipulé *in vivo* et/ou implanté *in vivo* et chargé avec au moins une composition pharmaceutique telle que définie selon l'invention pour son utilisation pour traiter ou prévenir une infection notamment bactérienne, en particulier ostéo-articulaire et touchant une articulation, un os, ou se développant au niveau d'un dispositif médical implanté.

Ce dispositif médical considéré peut être directement un dispositif de type implant dédié à être implanté, de manière pérenne ou non, *in vivo* mais également un pansement, une compresse ou une gaze dédiée à être mis en contact temporairement avec un organe interne ou un implant à des fins de traitement ou de prévention d'une infection bactérienne.

La présente invention concerne aussi l'utilisation d'une composition pharmaceutique telle que définie selon la présente invention, ou d'un dispositif médical interne tel que défini selon l'invention, pour une délivrance ciblée, locale et prolongée de l'antibiotique(s) au niveau d'une infection, en particulier bactérienne, de préférence ostéo-articulaire, touchant une articulation, un os, ou se développant au niveau d'un dispositif médical implanté ou au niveau d'un site potentiellement sujet à une telle infection, en particulier bactérienne, de préférence ostéo-articulaire.

### FIGURES

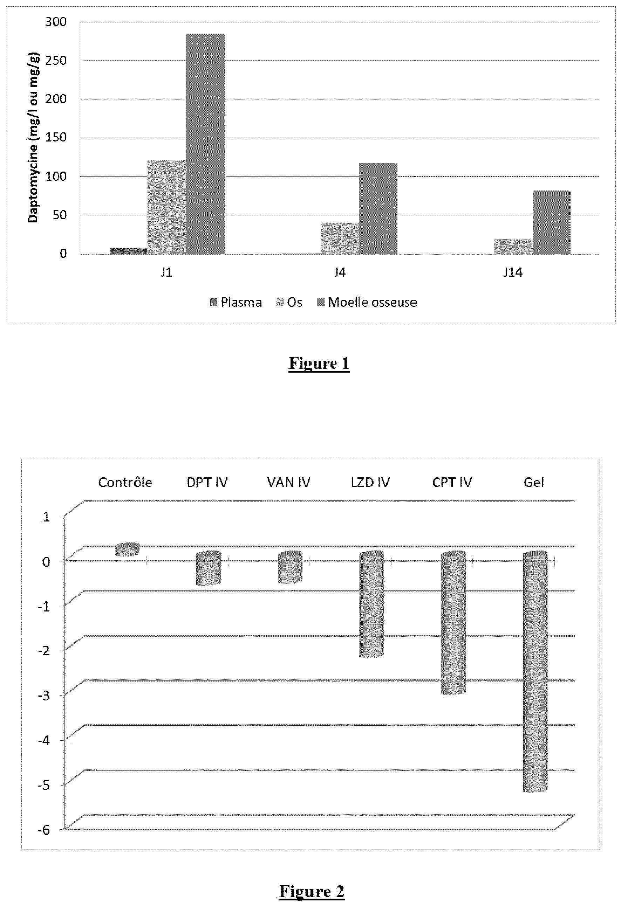

La figure 1 représente les concentrations en daptomycine mesurées 4 jours après traitement par une seule dose de 200 mg de la composition selon l'invention dans un modèle d'Ostéomyélite du Lapin à *Staphylococcus aureus* Méticilline Résistant (SAMR).
La figure 2 représente la détermination de la charge bactérienne (Δlog₁₀ CFU/g de tissu) au niveau de l'os 4 jours après traitement par une seule dose de 200 mg de la composition selon l'invention en comparaison avec d'autres antibiotiques administrés par voie intra-veineuse (IV) pendant 4 jours dans un modèle d'infection ostéo-articulaire expérimentale du Lapin à SAMR (*p<0,05 versus contrôle et autres groupes d'antibiotiques : LZD = Linézolide, DPT = Daptomycine, VAN = Vancomycine, CPT = Céftaroline).

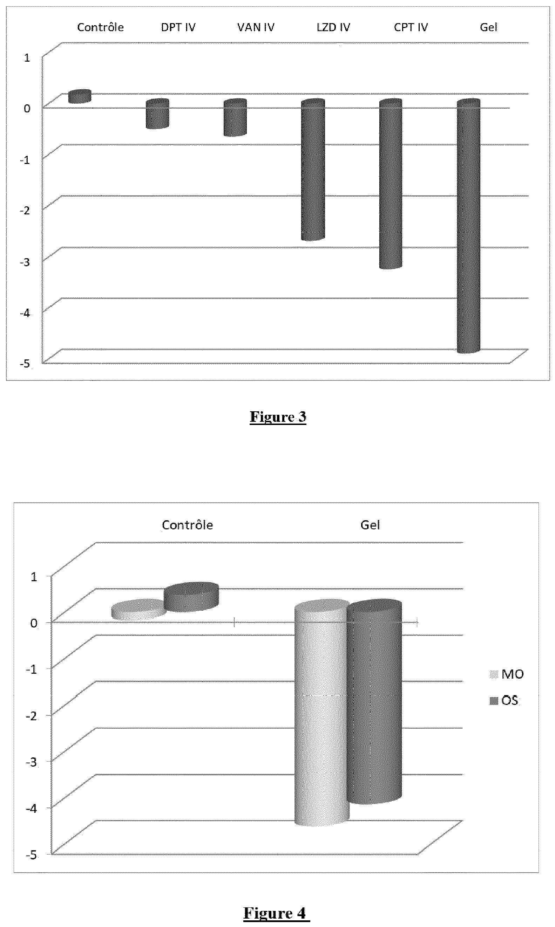

La figure 3 représente la détermination de la charge bactérienne (Δlog₁₀ CFU/g de tissu) au niveau de la moelle osseuse 4 jours après traitement par une seule dose de 200 mg de la composition selon l'invention en comparaison avec d'autres antibiotiques administrés par voie intra-veineuse (IV) pendant 4 jours dans un modèle d'infection ostéo-articulaire expérimentale du Lapin à SAMR (*p<0,05 versus contrôle et autres groupes d'antibiotiques : LZD = Linézolide, DPT = Daptomycine, VAN = Vancomycine, CPT = Céftaroline).
La figure 4 représente la détermination de la charge bactérienne (Δlog₁₀ CFU/g de tissu) au niveau de la moelle osseuse 14 jours après traitement par une seule dose de 200 mg de la composition selon l'invention en comparaison avec un groupe non traité dans un modèle d'infection ostéo-articulaire expérimentale du Lapin à SAMR (*p<0,05) versus contrôle.

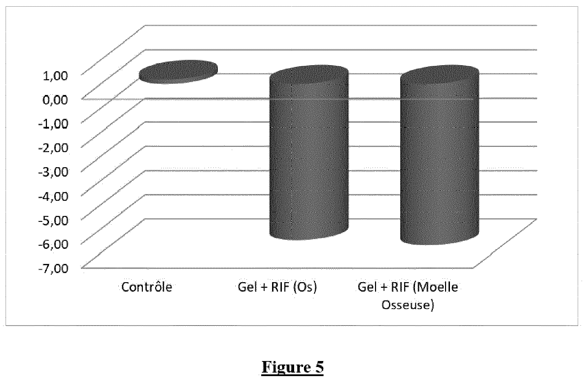

La figure 5 représente la détermination de la charge bactérienne (Δlog₁₀ CFU/g de tissu) au niveau de la moelle osseuse 4 jours après traitement par une seule dose de 200 mg de la composition selon l'invention associée à de la rifampicine (RIF) administrée par voie intra-musculaire (IM, 20 mg/kg toutes les 12 heures, pendant 4 jours), dans un modèle d'infection ostéo-articulaire expérimentale du Lapin à SARM.

### COMPOSITION

Comme énoncé précédemment, une composition selon l'invention se présente sous la forme d'un gel à au moins une température variant de 15 °C à 40 °C. Ainsi une composition selon l'invention est stabilisée à un état gélifié à une température variant au moins de la température ambiante à la température corporelle.

En particulier, une composition selon l'invention possède une viscosité entre 15 °C et 40 °C supérieure à 10 Pa.s, de préférence variant de 10 Pa.s à 10000 Pa.s, plus préférentiellement de 50 à 1000 Pa.s.

Cette viscosité peut être caractérisée à 25 °C avec un taux de cisaillement de 0,01 s⁻¹, par exemple à l'aide d'un rhéomètre tel que celui commercialisé par la Société Malvern sous la dénomination KINEXUS pro+.

Cet aspect gélifié est principalement ajusté *via* le choix de l'agent gélifiant polymérique associé.

Toutefois, il est clair que la concentration des nanocapsules et de l'actif, en l'occurrence l'antibiotique sous sa forme non encapsulée, va également contribuer à l'ajustement de cette viscosité.

La phase aqueuse gélifiée représente par exemple de 40 % à 90 % en poids, de préférence de 45 % à 85 % en poids, par rapport au poids total de la composition.

Cette phase aqueuse gélifiée comprend au moins un milieu aqueux, un gélifiant hydrophile polymérique, un principe actif, de préférence un antibiotique, et le cas échéant un polyol distinct dudit gélifiant hydrophile polymérique.

### a) Gélifiant hydrophile polymérique

Comme indiqué précédemment, la phase aqueuse est gélifiée avec au moins un gélifiant hydrophile polymérique.

Les gélifiants hydrophiles polymériques sont bien entendu choisis pour leur compatibilité avec une utilisation *in vivo* et leur inertie vis-à-vis d'une part de l'actif associé mais également des nanocapsules.

Conviennent tout particulièrement à ce titre les gélifiants hydrophiles polymériques choisis parmi les carboxyalkylcelluloses en C₁-C₂ possédant une viscosité supérieure à 1500 mPa.s, les copolymères tribloc constitués de poly(oxyde d'éthylène) et de poly(oxyde de propylène), les copolymères tribloc constitués de poly(éthylène glycol) et de poly(acide lactique-co-glycolique), et leurs mélanges.

Plus particulièrement, les copolymères tribloc constitués de poly(oxyde d'éthylène) et de poly(oxyde de propylène) peuvent être thermosensibles et présenter une température critique inférieure de solubilité (LCST) comprise entre 15 °C et 40 °C, et les copolymères tribloc constitués de poly(éthylène glycol) et de poly(acide lactique-co-glycolique) peuvent être thermosensibles et présenter une température critique inférieure de solubilité (LCST) comprise entre 15 °C et 40 °C.

Les carboxyalkylcelluloses convenant à l'invention sont les carboxyméthylcelluloses solubles dans l'eau, en particulier les carboxyméthylcelluloses et leurs sels de sodium.

Les carboxyméthylcelluloses haute viscosité (également nommée CMC HV) c'est-à-dire de viscosité au moins égale à 1500 mPa.s et notamment celles commercialisées par la société Sigma-Aldrich ou sous le nom commercial Calbiochem® par la société Merck Millipore et les carboxyméthylcelluloses ultra haute viscosité (également nommé CMC Ultra high viscosity) commercialisées par la société Sigma-Aldrich sont particulièrement adaptées pour la mise en oeuvre de l'invention.

Les copolymères tribloc constitués de poly(oxyde d'éthylène) (PEO) et de poly(oxyde de propylène) (PPO) convenant à l'invention peuvent être les copolymères de formule (PEO)x (PPO)y (PEO)z où x est compris entre 5 et 200, y est compris entre 5 et 100 et z est compris entre 5 et 200.

De préférence, les valeurs de x et z sont identiques.

Les composés dans lesquels x = 106, y = 69 et z = 106 sont très avantageusement utilisés.

Des copolymères tribloc particulièrement adaptés pour la mise en oeuvre de l'invention sont liquides à 4 °C et gélifiés à 37 °C. Ils sont ainsi hydrosolubles et thermosensibles. Ils possèdent de préférence un poids moléculaire compris entre 9500 et 15000 g/mol, en particulier entre 9800 et 14600 g/mol et plus particulièrement entre 9840 et 14600 g/mol et un taux d'oxyde d'éthylène compris entre 70,0 % et 80,0 %, en particulier entre 71,0 % et 75,0 %, et plus particulièrement entre 71,5 % et 74,9 %.

Les polymères triblocs constitués de poly(oxyde d'éthylène) (PEO) et de poly(oxyde de propylène) (PPO) commerciaux connus sous le nom de Pluronic® F127 (commercialisé par la société Sigma Aldrich, numéro de CAS 9003-11-6), Polaxamère® 407 ou P407 (commercialisé par la société BASF) ou encore le Pluronic® P123 commercialisé par BASF sont particulièrement adaptés pour la mise en oeuvre de l'invention.

Les copolymères tribloc constitués de poly(éthylène glycol) et de poly(acide lactique-co-glycolique) convenant à l'invention sont avantageusement des polymères tribloc de type PEG-PLGA-PEG, solubles dans les milieux aqueux et possédant un caractère thermosensible.

Selon un mode de réalisation préféré, les gélifiants hydrophiles polymériques compris dans la phase aqueuse gélifiée convenant à la présente invention sont choisis parmi les carboxyméthylcelluloses en C₁-C₂ possédant une viscosité supérieure à 1500 mPa.s, de préférence comprise entre 1500 mPa.s et 4500 mPa.s et/ou les copolymères tribloc poly(oxyde d'éthylène) et de poly(oxyde de propylène) présentant une température critique inférieure de solubilité (LCST) comprise entre 15 °C et 40 °C.

Bien entendu, la quantité de gélifiant polymérique est ajustée pour procurer la viscosité attendue à la composition selon l'invention.

Cet ajustement relève clairement des compétences de l'homme du métier.

En particulier, une composition selon l'invention peut comprendre de 1 % à 30 % en poids, de préférence de 2 % à 25 % en poids de gélifiant(s) hydrophile(s) polymérique(s), par rapport au poids de la phase aqueuse gélifiée.

Selon une variante préférée, une composition selon l'invention contient en outre un polyol.

### b) Polyols

On appelle « polyol », des molécules organiques comprenant au moins deux fonctions hydroxyles (-OH).

Ce polyol est distinct des polymères hydroxylés susceptibles d'être mis en oeuvre à titre de gélifiant.

La présence de ce polyol est tout particulièrement avantageuse pour ajuster le pH de la composition et/ou solubiliser les différents constituants.

De préférence, le polyol de la composition selon l'invention possède une chaîne hydrocarbonée linéaire saturée, ramifiée ou non ramifiée.

Avantageusement, le polyol comprend un nombre d'atomes de carbone allant de 2 à 20, de préférence de 2 à 12, encore mieux de 5 à 12, et comprend de 2 à 12, mieux de 2 à 8, et encore mieux de 2 à 6 fonctions hydroxyle.

Les polyols de la composition selon l'invention peuvent être choisis parmi le sorbitol (également nommé glucitol), le mannitol, le dulcitol, le maltitol, l'isomaltitol (également nommé isomalt), le xylitol, l'arabitol (également nommé lyxitol ou encore arabinitol), le ribitol (également nommé adonitol), le volemitol, et leurs mélanges, de préférence le sorbitol, le mannitol, le maltitol, et leurs mélanges, encore mieux le sorbitol.

Selon un mode de réalisation particulièrement préféré, le polyol est le sorbitol.

Une composition selon l'invention peut comprendre de 1 % à 30 % en poids, préférentiellement de 2 % à 20 % en poids, en polyol(s) par rapport à son poids total.

### c) Milieu aqueux

En ce qui concerne le milieu aqueux, il est formé d'eau, le cas échéant en mélange avec un ou plusieurs solvants annexes hydrosolubles.

Selon une variante de réalisation, ce milieu solvant est uniquement constituée d'eau.

### d) Nanocapsules lipidiques

Comme précisé ci-dessus, une composition selon l'invention contient en outre des nanocapsules.

Ces nanocapsules sont avantageusement présentes au sein de la phase aqueuse gélifiée.

Avantageusement, ces nanocapsules peuvent être présentes en une teneur variant de 2 % à 90 % en poids, voire de 5 % à 85 % en poids, voire de 10 % à 80 % en poids, voire de 10 % à 70 % en poids, par rapport au poids total de la composition.

En particulier, elles peuvent être mises en oeuvre dans un rapport pondéral nanocapsules/phase aqueuse gélifiée variant de 10/90 à 50/50.

Au sens de la présente invention, les nanocapsules lipidiques convenant à l'invention possèdent une architecture distincte des micelles, les micelles inverses ou les particules à architecture liposome.

Plus précisément, les nanocapsules lipidiques conformes à l'invention comprennent un noyau (ou un coeur) lipidique liquide ou semi-liquide à température ambiante, dans lequel au moins un antibiotique lipophile ou hydrophile est incorporé, ce noyau étant entouré (ou enveloppé) d'une enveloppe lipidique solide (donc rigide) à température ambiante.

L'expression « à température ambiante » signifie, dans le cadre de la présente invention, à une température comprise entre 15 °C et 25 °C.

L'expression « température corporelle » signifie, dans le cadre de la présente invention, à une température comprise entre 35 °C et 38 °C.

Un milieu « semi-liquide » doit être compris comme étant un milieu ayant une viscosité plus importante qu'un milieu liquide. Le milieu « semi-liquide » est obtenu par tous moyens adéquats, par exemple à partir du milieu sous forme liquide, de préférence par l'ajout de gélifiants. Néanmoins, le milieu semi-liquide peut également être constitué d'un composé ou combinaison de composés ayant une viscosité adéquate afin de se comporter comme un gel.

De préférence, les nanocapsules lipidiques convenant à l'invention ont une taille inférieure à 100 nm, de préférence d'une taille comprise entre 10 nm et 50 nm, avantageusement comprise entre 17 nm et 25 nm. Ces tailles peuvent être déterminées par diffusion dynamique de la lumière, spectroscopie à corrélation de photons, microscopie électronique à balayage, microscopie électronique à transmission en mode cryoscopique.

De préférence, les nanocapsules convenant à l'invention sont celles décrites et obtenues selon les procédés détaillés dans les documents WO2001064328, WO2009004214, WO2009001019, WO2010067037 et WO2015118496.

Selon une première variante, les nanocapsules lipidiques convenant à la présente invention sont des nanocapsules connues sous le nom commercial de « Soludots » de la société CARLINA Technologies. Elles comprennent un coeur lipidique à base de triglycérides à chaîne moyenne, des acides capryliques et/ou capriques, par exemple de Labrafac® WL 1349 de la société Gattefossé et/ou le Captex® 8000 de la société Abitec, qui est un tricaprylate de glycérol. Le coeur est stabilisé par une enveloppe solide constituée de phospholipides, par exemple une lécithine (ou une lécithine de soja connu sous le nom de Lipoid S75-3, de la société Lipoid GmbH, comprenant environ 69 % de phosphatidylcholine et environ 9 % de phosphatidyl éthanolamine ou encore le Lipoid S-100, de la société Lipoid GmbH) et d'un tensio-actif hydrophile non ionique, tel que du stéarate de polyéthylène glycol (2-hydroxystéarate de polyéthylèneglycol-660 commercialisé sous le nom Solutol® HS 15 (également appelé Kolliphor® HS15) par BASF).

De préférence, l'enveloppe solide enveloppant le coeur des nanocapsules lipidiques comprend, ou est essentiellement constituée, d'un tensio-actif lipophile qui est une phosphatidylcholine.

Selon une seconde variante, les nanocapsules lipidiques convenant à la présente invention sont également des « Soludots » telles que décrites précédemment, mais dont le coeur lipidique comprend en outre un additif, un excipient non ionique dispersible dans l'eau, par exemple le Labrasol® de la société Gattefossé, à base de composés d'esters de PEG et d'une fraction de glycérides.

De préférence, pour un principe actif lipophile et en particulier un antibiotique lipophile, le noyau lipidique liquide ou semi- liquide comprend, ou est essentiellement constitué, d'un corps gras liquide ou semi-liquide à température ambiante, par exemple un triglycéride ou un ester d'acide gras ou un de leurs mélanges.

Plus préférentiellement, le noyau lipidique liquide ou semi-liquide des nanocapsules lipidiques comprend, ou est essentiellement constitué, d'un ou plusieurs triglycérides, d'un ou plusieurs esters d'acide gras, ou un de leurs mélanges.

En revanche, pour un principe actif hydrophile et en particulier un antibiotique hydrophile, le principe actif doit être stabilisé, au niveau du noyau lipidique, sous la forme d'un système micellaire inverse. Par conséquent, le noyau lipidique liquide ou semi-liquide comprend une phase huileuse comprenant au moins un corps gras liquide ou semi-liquide à température ambiante, avantageusement au moins un triglycéride, un ester d'acide gras, ou un de leurs mélanges, et au moins un tensioactif.

De préférence, le(s) antibiotique(s) encapsulé(s) peu(ven)t représenter jusqu'à 30 % en poids des nanocapsules lipidiques convenant à l'invention, par rapport au poids total desdites nanocapsules lipidiques.

Selon une variante préférée, des nanocapsules lipidiques convenant à l'invention comprennent au moins :
- du stéarate de polyéthylène glycol, de préférence le 2-hydroxystéarate de polyéthylèneglycol-660 ;
- du tricaprylate de glycérol ;
- du chlorure de sodium ;
- un phospholipide choisi parmi la lécithine, la lécithine de soja et leurs mélanges, de préférence parmi la lécithine et la lécithine de soja ;
- un antibiotique, et en particulier la daptomycine.

Selon un mode plus particulièrement préféré, l'antibiotique compris dans le coeur des nanocapsules lipidiques convenant à l'invention est au moins une daptomycine.

Les nanocapsules lipidiques convenant à l'invention peuvent comprendre en outre un ou plusieurs autres principes actifs distinct(s) de(s) l'antibiotique(s) requis selon l'invention, par exemple une vitamine, un analgésique, un anti-inflammatoire, une hormone de croissance, un antiseptique, un hémostatique, et leurs mélanges.

### e) Antibiotiques

Lorsque le terme « antibiotique » est utilisé dans la présente description sans aucune autre précision par ailleurs, cela signifie qu'il peut s'agir aussi bien de l'antibiotique incorporé dans le coeur des nanocapsules que de celui compris dans la phase aqueuse gélifiée.

Une composition selon l'invention peut également être définie comme comprenant au moins un antibiotique libre et au moins un antibiotique encapsulé.

On entend désigner par :
- « antibiotique libre », l'antibiotique non encapsulé dans les nanocapsules et donc véhiculé directement dans la phase aqueuse gélifiée ;
- « antibiotique encapsulé », l'antibiotique uniquement présent dans le coeur des nanocapsules lipidiques.

Une composition peut comprendre de 5 % à 50 % en poids d'antibiotique(s), et de préférence de 10 % à 40 % en poids d'antibiotique(s), par rapport à son poids total.

La quantité d'antibiotique(s) contenue directement dans la phase aqueuse gélifiée (c'est-à-dire sans prendre en compte celle contenue dans les nanocapsules présentes dans cette même phase aqueuse gélifiée) peut notamment être d'au moins 50 mg/ml, de préférence comprise entre 50 et 500 mg/ml, plus préférentiellement comprise entre 75 et 400 mg/ml, encore plus préférentiellement comprise entre 100 et 300 mg/ml, voire mieux comprise entre 125 et 250 mg/ml par rapport au poids total de la phase aqueuse gélifiée.

La quantité d'antibiotique(s) contenu dans le coeur des nanocapsules peut représenter de 1 % à 20 % en poids, et de préférence de 2 % à 10 % en poids, du poids total des nanocapsules.

Un antibiotique convenant à la présente invention peut être choisi parmi :
- les lipopeptides, tels que par exemple la daptomycine,
- les glyco- ou lipo- glycopeptides, tels que par exemple la vancomycine, la telavancine, la téicoplanine,
- les polypeptides, tels que par exemple la colistine,
- les rifamycines, telles que par exemple la rifampicine, la rifabutine,
- les pénicillines, telles que par exemple la pénicilline G, la pénicilline V, l'amoxicilline, l'oxacilline,
- les céphalosporines, telles que par exemple le céflacor, la céfalexine, le céfuroxime, la céfixime,
- les pénèmes, tels que par exemple l'imipénème, le méropénème, le doripénème,
- les macrolides et apparentés, tels que par exemple l'érythromicyne A, la spiramycine, la clarithromycine, la roxithromycine, l'azithromycine, la midécamycine, la télithromycine, la virginiamycine, la pristinamycine, l'ansamycine,
- les aminoglycosides, tels que par exemple l'amikacine, la gentamicine, la tobramycine,
- les quinolones et leurs dérivés fluorés, tels que par exemple l'ofloxacine, la moxifloxacine, la ciprofloxacine, la lévofloxacine,
- les sulfonamides ou sulfamides, tels que par exemple le triméthoprime, le sulfaméthoxazole,
- les fusidanines, tel que l'acide fusidique,
- la fosfomycine,
- les oxazolidinones, tels que le linézolide, le tédizolide,
- les tétracyclines, telles que par exemple la doxycycline, la lymécycline, la méthacycline, la minocycline, et
- leurs mélanges.

Plus particulièrement, le ou lesdits antibiotiques sont choisis parmi la daptomycine, la rifampicine, la vancomycine, la colistine et leurs mélanges, et plus préférentiellement est au moins la daptomycine.

Selon une variante de réalisation, ledit ou lesdits antibiotiques compris dans le coeur desdites nanocapsules lipidiques et dans ladite phase aqueuse gélifiée sont identiques.

Selon une variante de réalisation préférée, le coeur desdites nanocapsules lipidiques d'une part et ladite phase aqueuse gélifiée d'autre part, contiennent tous deux au moins de la daptomycine.

Selon une autre variante de réalisation, ledit ou lesdits antibiotiques compris dans le coeur desdites nanocapsules lipidiques et dans ladite phase aqueuse gélifiée sont distincts.

Ainsi, le coeur des nanocapsules lipidiques peut comprendre la daptomycine et l'antibiotique compris dans ladite phase aqueuse gélifiée est différent de la daptomycine.

De même, la phase aqueuse gélifiée peut comprendre la daptomycine et l'antibiotique compris dans le coeur desdites nanocapsules lipidiques est différent de la daptomycine.

Une composition pharmaceutique selon l'invention peut également comprendre simultanément des nanocapsules lipidiques selon l'invention comprenant à titre d'unique antibiotique de la daptomycine et d'autres nanocapsules lipidiques comprenant au moins un antibiotique distinct de la daptomycine, en particulier choisi parmi les lipopeptides, les lipoglycopeptides, les polypeptides, les rifamycines, les pénicillines, les céphalosporines, les carbapénèmes, les macrolides, les aminoglycosides, les quinolones et leurs dérivés fluorés, les sulfonamides (ou sulfamides), les oxazolidinones, la fosfomycine, l'acide fusidique, les tétracyclines, et leurs mélanges.

### f) Autres composants de la composition

Bien entendu, une composition pharmaceutique selon l'invention peut comprendre en outre au moins un principe actif distinct(s) dudit/desdits antibiotique(s) requis selon l'invention.

Cet actif peut être présent directement dans la phase aqueuse gélifiée et/ou être présent dans la composition sous une forme encapsulée dans des nanocapsules distinctes de celles véhiculant ledit antibiotique.

A titre d'exemple, le ou les principes actifs additionnel(s) peu(ven)t être choisi(s) parmi des vitamines, des agents cicatrisants, des minéraux, des analgésiques, des anti-inflammatoires, des hormones de croissance, des antiseptiques, des hémostatiques, et leurs mélanges.

La composition pharmaceutique selon l'invention peut également comprendre en outre au moins un excipient et/ou additif et/ou solvant pharmaceutiquement acceptable.

Le ou les excipient(s) et/ou additif(s) pharmaceutiquement acceptables peuvent notamment être choisis en fonction du mode d'administration de la composition pharmaceutique.

### PROCEDE DE PREPARATION DE LA COMPOSITION SELON L'INVENTION

Une composition selon l'invention peut notamment être obtenue par simple mélange des nanocapsules conformes à l'invention des autres composants requis dans la composition selon l'invention. L'ordre d'introduction des différents composants n'est pas déterminant.

Une autre variante peut être de former au préalable la phase aqueuse par mélange dans le milieu aqueux des composants liquides et d'y introduire ensuite les nanocapsules.

Avantageusement, la préparation de la composition peut être effectuée à une température comprise entre 2 °C et 25 °C.

En ce qui concerne les nanocapsules, elles peuvent être préparées selon les protocoles décrits dans les exemples et détaillés dans les documents WO2001064328, WO2009004214, WO2009001019, WO2010067037 et WO2015118496.

### UTILISATIONS D'UNE COMPOSITION SELON L'INVENTION

Une composition pharmaceutique selon l'invention est avantageusement compatible avec les différents modes d'administration usuellement considérés dans le domaine thérapeutique.

Elle est en particulier avantageusement adaptée à une administration directement au niveau de l'articulation, de l'os, ou du dispositif médical implanté. Par exemple, son administration peut être réalisée par injection notamment *via* une seringue mais également par application d'un dépôt de composition en surface de l'organe ciblé ou d'un dispositif, implant ou autre, dédié à être mis en contact avec l'organe.

Ainsi, la composition selon l'invention est adaptée pour une utilisation pour traiter ou prévenir une infection bactérienne ostéo-articulaire touchant une articulation, un os, ou se développant au niveau d'un dispositif médical implanté.

La présente invention concerne également des dispositifs médicaux internes comprenant au moins une composition pharmaceutique selon l'invention.

Par « dispositif médical », au sens de la présente invention, on entend le terme générique désignant tout instrument, appareil, équipement, matière ou autre article, utilisé seul ou en association, à des fins thérapeutiques, pour le contrôle, le traitement ou l'atténuation d'une maladie.

Par « dispositif médical interne », on entend un dispositif médical adapté à une application directe au niveau de l'articulation, de l'os, ou du dispositif médical implanté.

La présente invention concerne encore un dispositif médical interne selon l'invention, pour son utilisation pour traiter ou prévenir une infection, en particulier bactérienne, de préférence ostéo-articulaire touchant une articulation, un os ou se développant au niveau d'un dispositif médical implanté, de préférence ledit dispositif médical interne étant alors représenté par un pansement, une compresse ou une gaze.

Un dispositif médical interne selon l'invention peut être notamment un implant, une prothèse, une vis, une plaque, un pansement, une compresse ou une gaze.

Le terme « implant » ou « prothèse » peut désigner un élément destiné à être introduit et à demeurer dans un organisme pour une durée plus ou moins longue. Il peut s'agir par exemple d'un implant actif (dont le fonctionnement nécessite une source d'énergie électrique), d'une puce électronique, d'un segment osseux, d'un os, d'une prothèse, y compris une prothèse visuelle ou une prothèse osseuse, notamment de la hanche ou du genou, d'un cathéter, d'une chambre implantable.

Pour ce qui concerne les pansements, les compresses ou les gazes, ils peuvent en particulier être dédiés à être mis au contact d'un os ou d'une articulation à des fins de prévention ou de traitement d'une infection bactérienne ostéo-articulaire, mais également au contact d'un dispositif interne implanté (i.e. prothèse, implant, plaque ou vis).

Par exemple, la composition pharmaceutique selon l'invention peut être appliquée sur au moins une partie de la surface du pansement, compresse ou gaze, destinée à entrer en contact avec le site de l'infection. Toutefois, elle peut également être incorporée au sein du pansement, compresse ou gaze, par tous moyens adéquats pour une diffusion vers le site de l'infection.

L'association d'une composition pharmaceutique selon l'invention au matériau constitutif d'un dispositif médical interne selon l'invention peut être réalisée avant et/ou simultanément à l'utilisation ou à l'implantation dans le corps dudit dispositif médical interne.

Les exemples et figures qui suivent sont présentés à titre illustratif et non limitatif du domaine de l'invention.

Les expressions « *compris entre* ... *et* ... », *« variant de* ... *à ...* » et « *allant de* ... *à* ... » doivent se comprendre bornes incluses, sauf si le contraire est spécifié.

### EXEMPLES

### EXEMPLE 1 : PREPARATION DE NANOCAPSULES CHARGEES EN DAPTOMYCINE

On réalise 2,548 g d'une émulsion contenant 56 mg de Lipoïd® S75-3 (Lécithine de soja commercialisée sous Lipoïd® S75-3 de la société Lipoid GmbH), 678 mg de Captex® 8000 lipophile (Tricaprylate de glycérol commercialisé sous Captex® 8000 de la société Abitec), 1,547 g de Kolliphor® HS15 (également nommé Solutol® HS15) de la société BASF), 71 mg de chlorure de sodium, 196 mg de daptomycine et 1,64 g d'une solution de sorbitol à 30 % dans de l'eau milli-Q.

L'ensemble des composants ci-dessus est réuni dans un même bêcher sous agitation magnétique. Un chauffage est appliqué jusqu'à atteindre une température de 85 °C. Toujours sous agitation magnétique, on laisse refroidir le système jusqu'à une température de 60 °C. Ce cycle (entre 85 °C et 60 °C) est réalisé jusqu'à ce que l'on observe l'annulation de la conductivité en fonction de la température. L'inversion de phase se produit au bout de trois cycles. Au dernier refroidissement, on effectue une trempe en jetant 12,5 ml d'eau distillée à 2 °C +/- 1 °C sur le mélange à 70 °C. Le système est alors maintenu sous agitation magnétique pendant 5 min.

Les particules obtenues dans les conditions précédemment décrites, après trois cycles de température, ont une taille moyenne de 43 +/- 7 nm. Leur polydispersité de taille est de 0,071. La taille des particules est mesurée par diffusion quasi-élastique de la lumière (« Dynamic Light Scattering » ou DLS). Une taille moyenne des particules de l'ordre de 50 nm est mesurée. Par ailleurs, une observation faite en microscopie de force atomique en mode contact (appareillage Park Scientic Instruments, Genève, Suisse) montre que les nanocapsules sont effectivement solides à la température de 25 °C.

### EXEMPLE 2 : PREPARATION D'UNE COMPOSITION SELON L'INVENTION

L'agent gélifiant polymérique retenu pour ces essais est de type copolymère tribloc constitué de poly(oxyde d'éthylène) et de poly(oxyde de propylène).

Les nanocapsules de l'exemple 1 sont mélangés sous agitation mécanique à 50 rpm à 4 °C, à 5,541 g de daptomycine, 1,64 g d'une solution de sorbitol à 0,3 mg/ml, 1,62 g d'une solution de sorbitol à 0,3 mg/ml dans l'eau de trempe et 490 mg du Pluronic® F127 (de la société BASF).

La viscosité de la composition de l'exemple 2 a en outre été évaluée à différents taux de cisaillement, à température ambiante (25 °C).

Ces caractéristiques sont résumées dans le tableau 1 ci-après.

**Tableau 1**

| Exemple 2 | Valeur (Pa.s) |
|---|---|
| Viscosité de la composition (à un taux de cisaillement de 0,00 s⁻¹) | 4700 |
| Viscosité de la composition (à un taux de cisaillement de 0,01 s⁻¹) | 2500 |
| Viscosité de la composition (à un taux de cisaillement de 0,10 s⁻¹) | 950 |
| Viscosité de la composition (à un taux de cisaillement de 10,00 s⁻¹) | 400 |
| Viscosité de la composition (à un taux de cisaillement de 100,00 s⁻¹) | 70 |

Les nanocapsules de l'exemple 2 ont en outre été caractérisées en taille (diamètre moyen, polydispersité) par diffusion dynamique de la lumière et leur potentiel zêta a été mesuré par des méthodes électrocinétiques. Pour la réalisation des mesures, les suspensions de nanocapsules ont été préparées en diluant les formules réalisées avec de l'eau ultra pure.

Le tableau 2 rend compte de leurs spécificités.

**Tableau 2**

| | |
|---|---|
| Taille hydrodynamique des nanocapsules (nm) | 22,3 |
| Potentiel Zêta des nanocapsules (mV) | -16,3 |

La détermination du taux de charges de la composition obtenue est réalisée par dosage HPLC-UV de la daptomycine. Pour ce faire, 50 mg de la composition ont été mélangés à 9,46 ml d'isopropanol, 4,151 ml de THF, 2,795 ml de PBS 0,2 M pH 5,5 et 3,225 ml d'eau Milli-Q. Puis, l'extrait a été filtré sur un filtre ayant une porosité de 0,22 µm et dilué avec du PBS 0,2 M pH 5,5 avant injection dans le système HPLC-UV. Le tableau 3 ci-après résume les résultats obtenus.

**Tableau 3**

| | Aspect | Concentration en daptomycine (mg/ml de composition) |
|---|---|---|
| Exemple 2 | Solution visqueuse jaune | 192,00 +/- 4,55 |

### EXEMPLE 3 : PREPARATION D'UNE COMPOSITION SELON L'INVENTION

L'agent gélifiant polymérique retenu pour ces essais est de type polymère de carboxyméthylcellulose.

Les nanocapsules de l'exemple 1 sont mélangés sous agitation mécanique à l'aide d'une pale en U à 200 rpm avec 20,50 g d'une solution de sorbitol à 0,3 mg/ml, et 48,49 g d'une solution de sorbitol pour dilution à 0,3 mg/ml sont ajoutés. Le carboxyméthylcellulose ultra haute viscosité, commercialisé par la société Sigma-Aldrich, et la daptomycine sont introduits. Le pourcentage de polymères par rapport à la masse de la phase aqueuse est de 3 %. Par la suite, le chauffage est interrompu et l'agitation mécanique maintenue pendant 2 heures. Les échantillons sont conservés à 4 °C.

La composition ainsi obtenue est d'aspect opaque et jaune et a une concentration en daptomycine de 187,55 mg/ml +/- 2,76.

### EXEMPLE 4 : TESTS D'EFFICACITE IN VIVO D'UNE COMPOSITION SELON L'INVENTION

Une dose de la composition selon l'exemple 2 a été injectée chez un modèle d'Ostéomyélite du Lapin à SARM par voie injectable *in situ.* Les dosages des tissus ont été réalisés à l'aide d'une HPLC, *in situ* (os et moelle osseuse) et au niveau du plasma, de la rate, du foie, des reins et des muscles 1 jour, 4 jours et 14 jours après injection, permettant d'obtenir la pharmacocinétique de la daptomycine. La synthèse des résultats (plasma, os et MO) est présentée en figure 1.

Les concentrations de la daptomycine sont très élevées 4 jours après l'unique administration, en particulier dans l'os et la moelle osseuse. De plus, cette concentration reste élevée même 14 jours après l'injection.

Afin de déterminer la charge bactérienne des tissus, 1 ml de la composition selon l'exemple 2 a été injectée *in situ* chez un modèle d'IOA (infection ostéo-articulaire) expérimentale du Lapin à SARM (*Staphylococcus aureus* Résistant à la Méticilline) pour une dose de 200 mg/ml en daptomycine (figures 2, 3 et 4).

Les charges bactériennes dans les deux compartiments (os et moelle osseuse) ont été significativement réduites par l'injection de la composition selon l'invention, 4 jours et 14 jours après injection, en comparaison avec d'autres antibiotiques de référence à des doses humanisées (Linézolide (LZD, 10 mg/kg/12h), Daptomycine (DPT, 6 mg/kg/j), Vancomycine (VAN, 100 mg/kg/j en perfusion continue) et Céftaroline (CPT, 10 mg/kg/12h) (p < 0,001 versus contrôles, VAN, LZD, CPT et DPT).

Enfin, la composition selon l'exemple 2 a été administrée par injection en local en combinaison avec un antibiotique, la rifampicine par voie IM à la dose de 20 mg/kg/12h.

Une diminution significative (p < 0,001 versus contrôle) de la charge bactérienne dans les deux compartiments osseux (os et moelle osseuse) est observée (figure 5).

## Revendications

1. Composition pharmaceutique stabilisée à un état gélifié à au moins une température variant de 15 °C à 40 °C, comprenant au moins :
- une phase aqueuse gélifiée avec au moins un gélifiant hydrophile polymérique,
- des nanocapsules lipidiques comprenant un coeur lipidique liquide ou semi-liquide à température ambiante enveloppé d'une enveloppe lipidique solide à température ambiante, lesdites phase aqueuse gélifiée et nanocapsules contenant au moins un antibiotique, identique ou différent, l'antibiotique dans ladite phase aqueuse y étant présent à l'état de soluté.

2. Composition pharmaceutique selon la revendication 1, **caractérisée en ce que** la viscosité de la composition est supérieure à 10 Pa.s, de préférence comprise entre 10 Pa.s et 10000 Pa.s, et plus préférentiellement comprise entre 50 Pa.s et 1000 Pa.s, à une température variant de 15 °C à 40 °C.

3. Composition pharmaceutique selon l'une quelconque des revendications précédentes, **caractérisée en ce que** ledit ou lesdits gélifiants hydrophiles polymériques sont choisis parmi les carboxyalkylcelluloses en C₁-C₂ possédant une viscosité supérieure à 1500 mPa.s, les copolymères tribloc constitués de poly(oxyde d'éthylène) et de poly(oxyde de propylène), en particulier thermosensibles et présentant une température critique inférieure de solubilité (LCST) comprise entre 15 °C et 40 °C, les copolymères tribloc constitués de poly(éthylène glycol) et de poly(acide lactique-co-glycolique), en particulier thermosensibles et présentant une température critique inférieure de solubilité (LCST) comprise entre 15 °C et 40 °C, et leurs mélanges.

4. Composition pharmaceutique selon l'une quelconque des revendications précédentes, **caractérisée en ce que** ledit gélifiant hydrophile polymérique est choisi parmi les carboxyméthylcelluloses en C₁-C₂ possédant une viscosité supérieure à 1500 mPa.s, de préférence comprise entre 1500 mPa.s et 4500 mPa.s, et/ou les copolymères tribloc poly(oxyde d'éthylène) et de poly(oxyde de propylène) présentant une température critique inférieure de solubilité (LCST) comprise entre 15 °C et 40 °C.

5. Composition pharmaceutique selon l'une quelconque des revendications précédentes, **caractérisée en ce que** ledit ou lesdits gélifiant(s) hydrophile(s) polymérique(s) est(sont) présent(s) en une quantité comprise entre 1 % et 30 % en poids, préférentiellement entre 2 % et 25 % en poids, par rapport au poids de la phase aqueuse gélifiée.

6. Composition pharmaceutique selon l'une quelconque des revendications précédentes, **caractérisée en ce que** ladite phase aqueuse gélifiée comprend en outre au moins un polyol, de préférence choisi parmi le sorbitol, le mannitol, le dulcitol, le maltitol, l'isomalitol, le xylitol, l'arabitol, le ribitol, le volemitol, et leurs mélanges, plus préférentiellement le sorbitol, le mannitol ou le maltitol, et leurs mélanges, encore plus préférentiellement le sorbitol.

7. Composition pharmaceutique selon l'une quelconque des revendications précédentes, **caractérisée en ce que** ledit ou lesdits polyol(s) est(sont) présent(s) en une quantité comprise entre 1 % et 30 % en poids, préférentiellement de 2 % à 20 % en poids, par rapport au poids total de la composition.

8. Composition selon l'une quelconque des revendications précédentes, dans laquelle le ou lesdits antibiotiques sont choisis parmi les lipopeptides, les glyco- ou lipoglycopeptides, les polypeptides, les rifamycines, les pénicillines, les céphalosporines, les pénèmes, les macrolides et apparentés, les aminoglycosides, les quinolones et leurs dérivés fluorés, les sulfonamides ou sulfamides, les fusidanines, la fosfomycine, les oxazolidinones, les tétracyclines, et leurs mélanges, de préférence le ou lesdits antibiotiques sont choisis parmi la daptomycine, la rifampicine, la vancomycine, la colistine et leurs mélanges, et plus préférentiellement est au moins la daptomycine.

9. Composition selon l'une quelconque des revendications précédentes, dans laquelle ledit ou lesdits antibiotiques compris dans le coeur desdites nanocapsules lipidiques et dans ladite phase aqueuse gélifiée sont identiques.

10. Composition selon l'une quelconque des revendications 1 à 8, dans laquelle ledit ou lesdits antibiotiques compris dans le coeur desdites nanocapsules lipidiques et dans ladite phase aqueuse gélifiée sont distincts.

11. Composition selon l'une quelconque des revendications 1 à 9, dans laquelle le coeur desdites nanocapsules lipidiques et la phase aqueuse gélifiée contiennent tous deux au moins de la daptomycine.

12. Composition selon l'une quelconque des revendications précédentes, dans laquelle le noyau lipidique liquide ou semi-liquide des nanocapsules lipidiques comprend, ou est essentiellement constitué, d'un ou plusieurs triglycérides, d'un ou plusieurs esters d'acide gras, ou un de leurs mélanges.

13. Composition selon l'une quelconque des revendications précédentes, dans laquelle l'enveloppe solide enveloppant le coeur des nanocapsules lipidiques comprend, ou est essentiellement constituée, d'un tensio-actif lipophile qui est une phosphatidylcholine.

14. Composition selon l'une quelconque des revendications précédentes, dans laquelle lesdites nanocapsules lipidiques ont une taille inférieure à 100 nm, de préférence comprise entre 10 nm et 50 nm, et mieux comprise entre 17 nm et 25 nm.

15. Composition selon l'une quelconque des revendications précédentes, dans laquelle les nanocapsules sont présentes en une teneur variant de 2 % à 90 % en poids, voire de 5 % à 85 % en poids, voire de 10 % à 80 % en poids, voire de 10 % à 70 % en poids, par rapport au poids total de la composition.

16. Composition selon l'une quelconque des revendications précédentes, ladite composition étant adaptée à une administration directement au niveau de l'articulation, de l'os, ou du dispositif médical implanté.

17. Composition selon l'une quelconque des revendications précédentes, pour son utilisation pour traiter ou prévenir une infection bactérienne ostéo-articulaire touchant une articulation, un os, ou se développant au niveau d'un dispositif médical implanté.

18. Dispositif médical interne comprenant une composition pharmaceutique selon l'une quelconque des revendications 1 à 17.

19. Dispositif médical interne selon la revendication précédente, ledit dispositif étant choisi parmi un implant, une prothèse, une vis, une plaque, un pansement, une compresse ou une gaze.

20. Dispositif médical interne selon la revendication 18 ou 19, pour son utilisation pour traiter ou prévenir une infection, en particulier bactérienne, de préférence ostéo-articulaire, touchant une articulation, un os, ou se développant au niveau d'un dispositif médical implanté, de préférence ledit dispositif médical interne étant alors représenté par un pansement, une compresse ou une gaze.

21. Utilisation d'une composition pharmaceutique selon l'une quelconque des revendications 1 à 17, ou d'un dispositif médical interne selon les revendications 18 à 20, pour une délivrance ciblée, locale et prolongée de l'antibiotique(s) au niveau d'une infection, en particulier bactérienne, de préférence ostéo-articulaire, touchant une articulation, un os, ou se développant au niveau d'un dispositif médical implanté ou au niveau d'un site potentiellement sujet à une telle infection.
